# EUROPEAN PATENT SPECIFICATION

(11) **EP 2 509 553 B1**
(45) Date of publication and mention of the grant of the patent: **31.08.2016**
(21) Application number: 09852111.5
(22) Date of filing: 10.12.2009
(51) Int. Cl.: A61F 13/15, B01D 53/62, B01D 53/73, C08K 3/26, A61F 13/84, A61L 15/18, B01J 20/04, B01J 20/28

(54) **ABSORBENT ARTICLES AS CARBON SINKS**
SAUGFÄHIGE ARTIKEL ALS KOHLENSTOFFSENKE
ARTICLES ABSORBANTS FAISANT OFFICE DE PUITS DE CARBONE

(43) Date of publication of application: 17.10.2012
(73) Proprietor: SCA Hygiene Products AB, 405 03 Göteborg (SE)
(72) Inventor: LAKSO, Elisabeth, S-444 45 Stenungsund (SE); STRIDFELDT, Chatrine, S-436 58 Hovås (SE)
(74) Representative: Valea AB
(86) International application number: PCT/SE2009/051399
(87) International publication number: WO 2011/071429

(56) References cited:
- WO-A1-96/09248
- WO-A1-96/09248
- WO-A1-2006/008242
- WO-A1-2007/071633
- WO-A1-2009/065031
- WO-A1-2009/155378
- WO-A1-2010/051458
- WO-A2-01/39807
- AU-B1- 2009 240 866
- US-A- 5 008 296
- US-A1- 2008 245 660
- US-A1- 2009 104 831

## Description

### TECHNICAL FIELD

The present invention relates to absorbent articles with a reduced carbon footprint, and methods for making such articles.

### BACKGROUND OF THE INVENTION

In recent years, the effect of manufacturing on the environment has become more and more relevant to consumers, governments and manufacturers. Particular attention has been paid to the total greenhouse gases emitted during a manufacturing process, in particular carbon dioxide.

The amount of carbon dioxide emitted, or absorbed during a process, during an event, or by a business, an individual or a product is often measured by its "carbon footprint". Tools such as Life Cycle Assessment (LCA) may be used to estimate the effect of a given product on CO₂ emissions. Cradle-to-grave is the full Life Cycle Assessment from extraction of raw material ('cradle') to use phase and disposal phase ('grave'). Cradle-to-gate is an assessment of a partial product life cycle from extraction of raw material ('cradle') to the factory gate. The term "gate" can apply to a number of points in a manufacturing process - e.g. finished material at the material producers' gate, or finished products at the gate of a further manufacturing process. Using these tools, efforts can be made to reduce the amount of atmospheric CO₂ emitted during a process, and to even adapt the process so that the overall result is a reduction in the amount of atmospheric CO₂.

Carbon dioxide from the atmosphere is converted into carbon-containing compounds during photosynthesis. Plants are therefore considered able to "lock" atmospheric carbon dioxide into their structure during their growth. However, subsequent decomposition of the plant matter - or incineration thereof - releases the locked-in carbon as carbon dioxide again. Absorbent products such as diapers typically comprise plant material (typically pulp fluff and cellulosic materials) where the carbon in these materials has originated from atmospheric CO₂. Upon disposal in landfill sites, or upon incineration, absorbent products decompose, and atmospheric CO₂ is released again.

WO 1996/009248 describes the use of atmospheric CO₂ as a source for papermaking fillers. A disposable diaper with improved degradability is disclosed in WO 01/39807 A2.

It would be useful to provide products of manufacture and manufacturing processes which are able to use atmospheric carbon dioxide as a source of carbon, but which do not necessarily release atmospheric CO₂ when said products decompose in landfill or through incineration. It would also be useful if the atmospheric CO₂ could be absorbed in a method other than photosynthesis, allowing a wider range of materials than plant-based materials to act as "sinks" for atmospheric CO₂.

### SUMMARY OF THE INVENTION

The present invention thus provides a disposable absorbent article such as a diaper, a sanitary napkin, a panty liner or a male or female incontinence article. The article comprises inorganic material. The inorganic material comprises carbon which is derived from atmospheric carbon dioxide (CO₂).

The inorganic material comprising carbon suitably has a ¹⁴C/¹²C ratio of 1 x 10⁻¹³ or greater, preferably 3 x 10⁻¹³ or greater, more preferably 5 x 10⁻¹³ or greater.
The inorganic material is suitably a carbonate salt, e.g. a carbonate salt of an alkali metal (Group 1), alkaline earth metal (Group 2) and mixtures thereof. The inorganic material may be calcium carbonate.

The absorbent article comprises a plastic film, and the inorganic material is located within the plastic film and/or the absorbent article comprises synthetic fibres, said inorganic material being located within the synthetic fibres.

The invention also provides a method for making a disposable absorbent article, said method comprising the steps of:
a. reacting atmospheric CO₂ with a metal salt (preferably a metal sulfate salt) in the presence of water to obtain a metal carbonate,
b. incorporating the metal carbonate obtained in step a. into said absorbent article.

The disposable absorbent article comprises a plastic film or synthetic fibres, the method thus includes the additional steps of:
b1. incorporating the metal carbonate into said plastic film or synthetic fibres
b2. incorporating said plastic film or synthetic fibres into said absorbent article.

The metal carbonate of the above method is suitably calcium carbonate.

### DEFINITIONS

By the term *"inorganic"* is meant a material which is not of plant or animal origin, and does not comprise organic materials such as cellulose, or fossil fuels such as oil etc. Inorganic materials are typically salts. Examples of inorganic materials include metal carbonates, cyanides, cyanates, carbides and thiocyanates.

An absorbent article is an article intended to be worn in the crotch region of a wearer, and which is used for the uptake and management of bodily exudate: e.g. blood, urine, feces etc. The article is worn closest to the skin, under the clothing of the wearer. Typically, absorbent articles are articles such as diapers, sanitary napkins, panty liners or male or female incontinence articles.

Carbon dioxide (CO₂) occurs naturally in the atmosphere in trace amount (less than 0.05%). "Atmospheric" carbon dioxide is used to mean carbon dioxide in gaseous form which comes directly from the earth's atmosphere. Additionally, "atmospheric" carbon dioxide can refer to carbon dioxide which is a waste product of industrial processes, e.g. obtained from the waste flues of other processes and recycled.

### BRIEF DESCRIPTION OF THE DRAWINGS

The invention will now be described in more detail with reference to the appended schematic drawings, in which
- Figure 1: illustrates an absorbent article according to the invention, being a diaper.
- Figure 2: is a cross-sectional view along the line II-II in Figure 1.

### DETAILED DESCRIPTION OF PREFERRED EMBODIMENTS

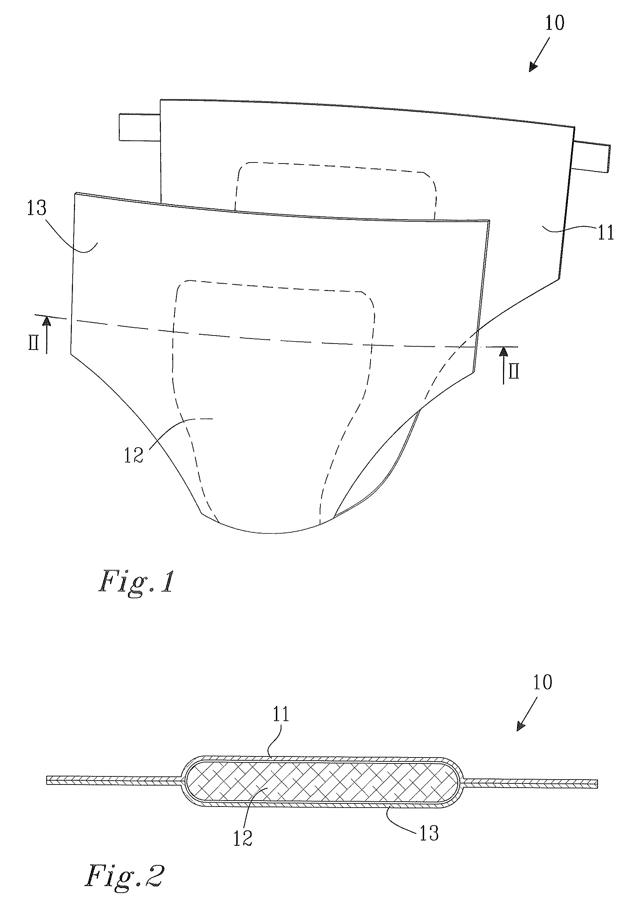

Figure 1 shows an absorbent article 10 according to the invention, in the form of a diaper. Although the invention is described with reference to a diaper, it is equally relevant to other absorbent articles, such as e.g. sanitary napkins, panty liners or male or female incontinence articles. As stated above, the term "absorbent article" refers to products that are worn against the skin of the wearer to absorb and contain body exudates, like urine, faeces and menstrual fluid. The present invention mainly relates to disposable absorbent articles, which means articles that are not intended to be laundered or otherwise restored or reused as an absorbent article after use.

As shown in Figure 1, and in cross-section in Figure 2, the absorbent article 10 typically comprises a liquid-pervious topsheet 11 which lies closest to the wearer when the article 10 is being worn. An absorbent body 12 typically lies under the topsheet 11. A liquid-impervious backsheet 13 is usually located between the absorbent core 12 and the wearer's garments. Usually, absorbent articles 10 comprise all three of these components; however, certain absorbent articles 10 may lack one of these components.

The liquid-pervious topsheet 11 lies closest to the wearer's skin, and makes contact therewith. It also allows bodily exudate to pass through to the underlying absorbent body 12. The materials suited as topsheet 11 should therefore be soft and non-irritating to the skin and intended to be readily penetrated by body fluid, e.g. urine or menstrual fluid. The topsheet 11 can consist of a nonwoven material, e g spunbond, meltblown, carded, hydroentangled, wetlaid etc. Suitable nonwoven materials can be composed of natural fibers, such as woodpulp or cotton fibres, manmade fibres, such as polyester, polyethylene, polypropylene, viscose etc. or from a mixture of natural and manmade fibres. In certain circumstances, topsheet 11 may comprise a plastic film, which is perforated to allow the passage of liquid. The topsheet 11 may further be composed of tow fibres, which may be bonded to each other in a bonding pattern, as e.g. disclosed in EP-A-1 035 818. Further examples of materials suitable for topsheet 11 are porous foams, apertured plastic films etc. The topsheet 11 may be different in different parts of the absorbent article.

The liquid-impervious backsheet 13 lies on the garment-facing surface of the absorbent body 12 and acts to prevent bodily exudate from leaking from the absorbent body 12 and staining the wearer's garments. The liquid-impervious backsheet 13 covering the absorbent body 12 on the garment-facing side is therefore of a liquid impervious material, such as a thin plastic film, e.g. a polyethylene or polypropylene film, a nonwoven material coated with a liquid impervious material, a hydrophobic nonwoven material which resists liquid penetration or a laminate of one or more plastic films with one or more nonwoven materials. For an improved feel, the liquid-impervious backsheet 13 preferably comprises a soft nonwoven material on the garment-facing surface thereof. The liquid-impervious backsheet 13 could be breathable so as to allow vapour to escape from the absorbent body 12, while still preventing liquids from passing therethrough. Examples of breathable liquid-impervious backsheets 13 are porous polymeric films, nonwoven laminates from spunbond and meltblown layers, laminates from porous polymeric films and nonwovens.

The absorbent body 12 acts to accept and retain bodily exudate from the wearer. It therefore comprises absorbent material. The absorbent body 12 can be of any conventional kind. Examples of commonly occurring absorbent materials are cellulosic fluff pulp, tissue layers, highly absorbent polymers (so called superabsorbents), absorbent foam materials, absorbent nonwoven materials or the like. It is common to combine cellulosic fluff pulp with superabsorbents in an absorbent body 12. The thin absorbent bodies, which are common in for example baby diapers and incontinence guards, often comprise a compressed mixed or layered structure of cellulosic fluff pulp and superabsorbent. The size and absorbent capacity of the absorbent body 12 may be varied to be suited for different uses such as for infants or for incontinent adults.

The absorbent body 12 may comprise one or more layers which may be selected to improve the handling of bodily waste. Such layers are designed to receive a large amount of liquid in a short space of time and distribute it evenly across the absorbent body 12. They may include so-called transfer, distribution, surge or acquisition layers, and are usually located between the topsheet 11 and the absorbent body 12 and/or between the absorbent body 12 and the backsheet 13.

The topsheet 11 and backsheet 13 generally have a similar extension in the X-Y plane, while the absorbent body 12 usually has an extension which is somewhat smaller. The topsheet 11 and backsheet 13 are joined to one another around the periphery of the absorbent body 12, so that the absorbent body 12 is enclosed within the envelope formed by the topsheet 11 and the backsheet 13. The absorbent body 12 is at least located in the crotch portion of the absorbent article 10, and may also extend somewhat into the front and rear portions. The topsheet 11, absorbent body 12 and backsheet 13 may be joined to one another by any means common in the art, e.g. ultrasonic welding, thermal welding or gluing.

The absorbent article 10 comprises inorganic material. The inorganic material may have various functions; e.g. to absorb liquid or odours, to provide bulk to the absorbent article and the materials thereof, or to provide breathability in that the inorganic material facilitates the formation of a porous structure.

The inorganic material comprises carbon. Most suitably, the carbon is present as a carbonate anion (CO₃²⁻). Other inorganic materials comprising carbon include e.g. cyanides (CN⁻), cyanates (OCN⁻), carbides, thiocyanates (SCN⁻).

Suitably, the inorganic material is a salt - i.e. a material consisting of cations and anions in a suitable ratio so that the overall charge is zero. For instance, the inorganic material may consist of carbonate salts, e.g. the carbonate salts of alkali metals (Group 1: e.g. Li, Na, K) and alkaline earth metals (Group 2: e.g. Mg, Ca) and mixtures thereof. Most suitably, the inorganic material is calcium carbonate (CaCO₃).

The carbon in the inorganic material is derived from atmospheric carbon dioxide (CO₂). In other words, chemical processes are carried out in which atmospheric carbon dioxide is reacted with other suitable reagents to form the inorganic material. By the term *"derived from",* is meant *"substantially immediately derived from".* That is, the term is not meant to include inorganic materials which occur naturally, and which may have - at some point in their geological history - incorporated carbon dioxide from the atmosphere as part of the natural carbon cycle. For instance, the inorganic material according to the invention is derived from atmospheric carbon dioxide (CO₂) within the preceding 20 years, preferably within the preceding 10 years.

In that the carbon in the inorganic material is derived from atmospheric carbon dioxide, carbon dioxide is removed from the atmosphere during the manufacture of the inorganic material, and thus, the manufacture of the absorbent articles 10. Therefore, during disposal of the absorbent articles, particularly in landfill, carbon which has its origins in atmospheric CO₂ is sequestered from the atmosphere and trapped in inorganic form.

There is a possibility to use CaO to recycle the CO₂ that is released when CaCO₃ is incinerated. Firstly, calcium oxide is warmed in the presence of atmospheric CO₂ to obtain calcium carbonate, thus extracting CO₂ from the atmosphere. This step requires a process temperature of around 400°C. The high process temperatures could be obtained via the heat from incineration or using solar cells. That would reduce the emission from the CaCO₃ when incinerated and produce new CaCO₃ that could be used. The CO₂ eq/kg obtained for the materials of the present invention would then be lower.

The absorbent articles 10 of the invention can be distinguished from known absorbent articles by analysing the ratio of carbon isotopes in the inorganic material (in a process similar to "carbon-dating"). The isotope of interest is ¹⁴C, which is often created in the upper layers of the earth's atmosphere. ¹⁴C decays radioactively. As ¹⁴C is constantly being generated in the atmosphere, the ratio of ¹⁴C to ¹²C in the atmosphere is relatively constant. However, when ¹⁴C is trapped in rocks or minerals, or fossil fuels such as oil, the amount of ¹⁴C gradually falls. The invention thus provides an absorbent article, in which the inorganic material comprising carbon has a ¹⁴C/¹²C ratio of 1 x 10⁻¹³ or greater, preferably 3 x 10⁻¹³ or greater, more preferably 5 x 10⁻¹³ or greater. Further details of the ¹⁴C/¹²C ratio, and suitable methods for its determination are discussed in US2007/0219521 and references cited therein.

When the inorganic material is calcium carbonate, a suitable method for the manufacture of this material from atmospheric CO₂ may be found in WO1996/009248. The reaction formula to make this CaCO₃ is CaSO₄ + H₂O + CO₂ (atm) → CaCO₃ + H₂SO₄

The inorganic material of the invention may be located in one or more components of the absorbent article 10 set out above. That is, it may be located in the topsheet 11, the absorbent body 12 or the backsheet 13, or more than one of these components. The inorganic material is usually present in particulate form.

The inorganic material of the invention may be located within a plastic film. Suitable polymers for making the plastic films include polyalkenes (e.g. polyethylene, polypropylene, or mixtures thereof), polyesters and polyamides, or mixtures thereof. Suitably, the polymer may come from a renewable source (so-called bio-polymers). Such bio-polymers would have a further positive effect on the calculation of CO₂ equivalents emitted during the disposal or incineration of the materials and products of the invention. Plastic films comprising inorganic material are known in the art, e.g. US6576809, JP2000136254. Such films are typically manufactured by adding the inorganic material to the molten polymer, prior to stretching or blowing the polymer into a film. The inorganic material in such films provides porosity to the film. The inorganic material may be present in 30-70 weight % in the films of the prior art. According to the invention, the inorganic material may be present in the plastic film in 2-80 weight %, preferably 5-70 weight %. If the inorganic material is located within a plastic film, this plastic film may be present in the topsheet 11 or the backsheet 13 of the article 10, preferably the backsheet 13. The invention therefore provides an absorbent article 10, wherein the absorbent article 10 comprises a plastic film, said inorganic material being located within the plastic film. Even more preferably, the backsheet 13 of the absorbent article 10 of the invention comprises a laminate, in which the plastic film of the invention is one of the layers.

The inorganic material of the invention may be located within synthetic fibres. Suitable polymers for making the synthetic fibres include polyalkenes (e.g. polyethylene, polypropylene, or mixtures thereof), polyesters and polyamides, or mixtures thereof. As for the film, above, the polymer of the synthetic fibres may come from a renewable source (so-called bio-polymers). The fibres may be monocomponent, bicomponent or multicomponent fibres. The fibres may be continuous fibres or staple fibres. Synthetic fibres comprising inorganic material are known in the art, e.g. US 20090104831, US 4341213. The inorganic material in such fibres provides porosity and other properties to the fibres. Inorganic material may be added to the polymer from which the fibres are made, prior to the fibres being drawn or blown. Inorganic material may be present in the synthetic fibres of the invention in 2 - 40 weight %, preferably 5 - 25 weight %. The synthetic fibres comprising the inorganic material of the invention may be formed into a nonwoven layer (e.g. as part of the topsheet 11, backsheet 13 or another layer), or may be loosely arranged as part of the absorbent body 12. The invention therefore provides an absorbent article 10, wherein the absorbent article 10 comprises synthetic fibres, and the inorganic material being located within the synthetic fibres.

Alternatively, the inorganic material of the invention may be located within a nonwoven layer. The inorganic material of the invention may be used in conjunction with any known nonwoven technique and any fibre compositions known in the art. For instance, the inorganic material may be incorporated in the structure of the nonwoven layer during its manufacture; e.g. by mixing inorganic material into the fibre mixture from which the nonwoven layer is made, or by adding the inorganic material to an existing nonwoven layer. Rather than being located within the synthetic fibres themselves, as above, the inorganic material in this case is trapped between individual fibres. Binder material may be used to incorporate the inorganic material into a nonwoven layer.

The inorganic material of the invention may be present in the absorbent article 10 in one or more of the components of the absorbent body 12. For example, the inorganic material of the invention may be present in the superabsorbent polymer (SAP) which is a key component of the absorbent bodies 12 of modern absorbent articles 10. Alternatively, the inorganic material may be bound elsewhere within the absorbent body 12 of the absorbent article 10.

As a further alternative, the inorganic material of the invention may simply be loose inside the absorbent article 10. It is therefore present within the envelope formed by the topsheet 11 and the backsheet 13.

The present invention also provides a method for making an absorbent article 10. The method comprises the steps of:
a. reacting atmospheric CO₂ with a metal salt in the presence of water to obtain a metal carbonate,
b. incorporating the metal carbonate obtained in step a. into said absorbent article 10.

Suitably, the metal salt is a metal sulfate salt. For the case of a metal sulfate salt, step a. of this method is discussed in more detail in WO1996/009248. In the particular case where the absorbent article 10 comprises a plastic film or synthetic fibres, the method includes the additional steps of:
b1. incorporating the metal carbonate into said plastic film or synthetic fibres
b2. incorporating said plastic film or synthetic fibres into said absorbent article 10.

All features of the absorbent article 10, the components thereof and the inorganic material described above are equally applicable to the methods of the present invention. In particular, the metal carbonate used in the above methods may be calcium carbonate. Plastic film or synthetic fibres may be incorporated into any suitable component of the absorbent article 10. In particular, the plastic film may be incorporated into the backsheet 13 of the absorbent article 10.

The invention has been described in relation to a number of embodiments and figures. However, the invention should not be considered as being limited to these embodiments, but rather, the scope of the invention is defined by the appended claims.

### CARBON FOOTPRINT CALCULATIONS

Calculations have been carried out for CaCO₃ manufactured from CaSO₄ and atmospheric CO₂ according to the equation:

CaSO₄ + H₂O + CO₂ → CaCO₃ + H₂SO₄

1 mole CO₂ gives 1 mole CaCO₃. The molecular weight is 80g/mole for CaCO₃ and 44 g/mole for CO₂.

0.55 kg CO₂ is required to produce 1 kg CaCO₃, and in the calculations, it is assumed that production of 1 kg CaCO₃ consumes 0.5 kg CO₂ eq. Consumption has been approximated to 0.5kg CO₂ eq., as there is the chance that small amounts of CO₂ are generated in the process.

As the CaSO₄ is a waste product from other industrial processes, no CO₂ contribution from producing this material is added.

Comparisons are made with CaCO₃ which is mined from the ground. The mining process has itself a certain carbon footprint.

The other values are from the following references:
Production of PP and PE : Plastics Europe (March 2005) I Boustead, Eco-profiles of the European Plastics Industry,
Production of CaCO3: Ecoinvent Data v.2.0 (2007), Kellenberger D. et al. Life Cycle Inventories of Building Products., Ecoinvent Report no. 7.
*Waste Handling:*
   Ecoinvent Data v.2.0 (2007), Doka G. Life Cycle Inventories of Waste Treatment Services, Ecoinvent Report no. 13.

### Examples of comparative calculations

### Example 1- polymer granules

CO₂ equivalents were estimated for the production of polymer granules. In other words, the "gate" in this example is considered as the production of finished polymer granules.

It is important to take into consideration that the illustrated values for the polymers lack the CO₂ contribution for converting the polymer granules to film or nonwoven. The conversion of the polymer granules to films and fibres, and product manufacture, can give different CO₂ equivalents depending on how efficient the converting lines are and the energy source used for production. Both the extrusion to film and spinning to fibres to production of NW are very dependent on the production unit, the technology and the energy source (which differ from country to country). In nonwoven production, energy consumption gives a CO₂ contribution of 0.7-1.4. Extrusion of film probably gives a somewhat lower contribution as the process temperature is lower. This value has therefore not been added, as it is assumed that all the films compared will have approximately the same values, if produced in the same location with the same equipment. There will be an effect in the relative values for different films in percent reduction of CO₂ equivalents.

Cradle to gate calculations are based on the production of polymer granules. Different waste options; incineration and landfill have been studied. These data are shown in Table 1

**Table 1: Calculated CO₂ equivalents for the cradle-to-gate production of various polymer granules and waste options**

| | *production CO₂ eq*/*kg* | *incineration CO*₂ *eq*/*kg* | *landfill CO₂ eqlkg* |
|---|---|---|---|
| PE granulates | 2.06 | 3.01 | 0.11 |
| PP granulates | 1.94 | 3.01 | 0.11 |
| Mined CaCO₃ | 0.02 | 0.44 | 0.11 |
| | | | |
| CaCO₃ from atmospheric CO₂ | -0.50 | 0.44 | 0.11 |

### Example 2 - Polymer films

Cradle-to-gate and cradle-to-grave estimations were then made for the production of film, in which pure PE granules are compared with PE granules filled with 50% of CaCO₃ and the PE granules of the invention filled with 50% CaCO₃ derived from atmospheric CO₂. It is estimated that 50% filler will be reasonable from a process point of view.

Estimations were made for 100% incineration, 100% landfill and 70% landfill/30% incineration (the typical waste disposal ratio in Europe). The results are shown in Table 2. The surprising effect is that the reduction in carbon footprint is higher than the percentage of filler with the 70/30 option, a reduction of 55% compared with PE. This is due to the CO₂ being "locked" in landfill. There is a CO₂ reduction of 16% compared to a PE film filled with CaCO₃ which has been mined from a geological source.

**Table 2: Exemplified calculations for CO₂ equivalents for the cradle-to-gate production of various films**

| | **Cradle to gate** | **Cradle to grave incineration** | **Cradle to grave landfill** | **Cradle to grave 70/30 landf/inc** |
|---|---|---|---|---|
| Film | CO₂ eq/kg | | | |
| PE | 2.06 | 5.06 | 2.17 | 3.04 |
| PE filled with 50% mined CaCO₃ | 1.04 | 2.76 | 1.15 | 1.63 |
| PE filled with 50% CaCO₃ from atmospheric CO₂ | 0.78 | 2.50 | 0.89 | 1.37 |
| **Reduction of CO₂ equivalents for PE filled with 50% CaCO₃ from atmospheric CO₂ compared to** | % | | | |
| PE | 62 | 51 | 59 | 55 |
| PE filled with 50% mined CaC03 | 25 | 9 | 23 | 16 |

### Example 3 - polymer fibres

Similar estimations were made for polymer fibres filled with CaCO₃. Values were estimated for polypropylene (PP) fibres. The data is shown in Table 3. It is estimated that 20% filler will be reasonable from a process point of view. An estimated 20% reduction in CO₂ equivalents per kg can be obtained using 20% CaCO₃ filler according to the invention as compared to pure PP fibres.

**Table 3: Exemplified calculations for CO₂ equivalents for the cradle-to-gate production of various fibres**

| **Fibres** | **Cradle to gate CO₂ eq/kg** | **Cradle to grave incineration** | **Cradle to grave landfill** | **Cradle to grave 70/30 landf/inc** |
|---|---|---|---|---|
| PP | 1.94 | 4.94 | 2.04 | 2.84 |
| PP filled with 20% mined CaCO₃ | 1.56 | 4.05 | 1.67 | 2.38 |
| PP filled with 20% CaCO₃ from atmospheric CO₂ | 1.45 | 3.94 | 1.56 | 2.27 |
| **Reduction of CO₂ equivalents for PP filled with 20% CaCO₃ from atmospheric CO₂ compared to** | % | | | |
| PP | 25 | 20 | 24 | 20 |
| PP filled with 20% mined | | | | |
| CaCO₃ | 7 | 3 | 6 | 4 |

Although the above calculations have been made on the basis of CaCO₃, the invention is of course not limited to CaCO₃. Similar calculations may be made for other inorganic materials produced from atmospheric CO₂.

## Claims

1. A disposable absorbent article (10) such as a diaper, a sanitary napkin, a panty liner or a male or female incontinence article, said article (10) comprising a plastic film and/or synthetic fibres, said plastic film and/or said synthetic fibres comprising inorganic material, **characterized in that** said inorganic material comprises carbon being derived from atmospheric carbon dioxide (CO₂) and having a ¹⁴C/¹²C ratio of 1 x 10⁻¹³ or greater, preferably 3 x 10⁻¹³ or greater, more preferably 5 x 10⁻¹³ or greater and wherein said inorganic material is a carbonate salt of an alkali metal (Group 1) selected among lithium (Li), sodium (Na) and potassium (K) or a carbonate salt of an alkaline earth metal (Group 2) selected among magnesium (Mg) and calcium (Ca), and mixtures thereof.

2. An absorbent article (10) according to claim 1, wherein the inorganic material is calcium carbonate.

3. A method for masking a disposable absorbent article (10), said absorbent article comprising a plastic film and/or synthetic fibres, said method comprising the steps of:
a. reacting atmospheric CO₂ having a ¹⁴C/¹²C ratio of 1 x 10⁻¹³or greater, preferably 3 x 10⁻¹³ or greater, more preferably 5 x 10⁻¹³ or greater with a metal salt being the salt of an alkali metal (Group 1) selected among lithium (Li), sodium (Na) and potassium (K) or a salt of an alkaline earth metal (Group 2) selected among magnesium (Mg) and calcium (Ca) in the presence of water to obtain a metal carbonate being an alkali metal carbonate or an alkaline earth metal carbonate,
b1. incorporating the metal carbonate into said plastic film or synthetic fibres
b2. incorporating said plastic film or synthetic fibres into said absorbent article.

4. A method according to claim 3 wherein the metal salt is a metal sulfate salt.

5. A method according to any of claims 3-4 wherein the metal carbonate is calcium carbonate.

## Patentansprüche

1. Saugfähiger Einwegartikel (10), zum Beispiel eine Windel, eine Damenbinde, eine Slipeinlage oder ein Inkontinenzartikel für Männer oder Frauen, wobei der Artikel (10) eine Kunststofffolie und/oder synthetische Fasern umfasst, wobei die Kunststofffolie und/oder die synthetischen Fasern ein anorganisches Material umfassen,
**dadurch gekennzeichnet, dass**
das anorganische Material Kohlenstoff umfasst, der von atmosphärischen Kohlenstoffdioxid (CO₂) stammt und ein ¹⁴C/¹²C-Verhältnis von 1 x 10⁻¹³ oder größer, bevorzugt 3 x 10⁻¹³ oder größer, mehr bevorzugt 5 x 10⁻¹³ oder größer, aufweist, und wobei das anorganische Material ein Carbonatsalz eines Alkalimetalls (Gruppe 1), ausgewählt aus Lithium (Li), Natrium (Na) und Kalium (K), oder ein Carbonatsalz eines Erdalkalimetalls (Gruppe 2), ausgewählt aus Magnesium (Mg) und Calcium (Ca), und Mischungen davon ist.

2. Saugfähiger Artikel (1) gemäß Anspruch 1, worin das anorganische Material Calciumcarbonat ist.

3. Verfahren zur Herstellung eines saugfähigen Einwegartikels (10), wobei der saugfähige Artikel eine Kunststofffolie und/oder synthetische Fasern umfasst, wobei das Verfahren die Schritte umfasst:
a. das Umsetzten von atmosphärischem CO₂ mit einem ¹⁴C/¹²C-Verhältnis von 1 x 10⁻¹³ oder größer, bevorzugt 3 x 10⁻¹³ oder größer, mehr bevorzugt 5 x 10⁻¹³ oder größer, mit einem Metallsalz, das ein Salz eines Alkalimetalls (Gruppe 1), ausgewählt aus Lithium (Li), Natrium (Na) und Kalium (K), oder ein Salz eines Erdalkalimetalls (Gruppe 2), ausgewählt aus Magnesium (Mg) und Calcium (Ca), ist, in Gegenwart von Wasser, um ein Metallcarbonat zu erhalten, das ein Alkalimetallcarbonat oder ein Erdalkalimetallcarbonat ist,
b1. das Einarbeiten des Metallcarbonats in die Kunststofffolie oder die synthetischen Fasern,
b2. das Einarbeiten der Kunststofffolie oder der synthetischen Fasern in den absorbierenden Artikel.

4. Verfahren gemäß Anspruch 3, bei dem das Metallsalz ein Metallsulfatsalz ist.

5. Verfahren gemäß einem der Ansprüche 3 bis 4, bei dem das Metallcarbonat Calciumcarbonat ist.

## Revendications

1. Article absorbant jetable (10) tel qu'une couche, une serviette hygiénique, un protège-slip ou un article d'incontinence chez l'homme ou la femme, ledit article (10) comprenant un film en matière plastique et/ou des fibres synthétiques, ledit film en matière plastique et/ou lesdites fibres synthétiques comprenant une matière inorganique, **caractérisé en ce que** ladite matière inorganique comprend du carbone qui est dérivé du dioxyde de carbone atmosphérique (CO₂) et présente un rapport ¹⁴C/¹²C supérieur ou égal à 1 x 10⁻¹³, de préférence supérieur ou égal à 3 x 10⁻¹³, de manière davantage préférée supérieur ou égal à 5 x 10⁻¹³ et dans lequel ladite matière inorganique est un sel de type carbonate d'un métal alcalin (Groupe 1) choisi parmi du lithium (Li), du sodium (Na) et du potassium (K) ou un sel de type carbonate d'un métal alcalino-terreux (Groupe 2) choisi parmi du magnésium (Mg) et du calcium (Ca), et des mélanges de ceux-ci.

2. Article absorbant (10) selon la revendication 1, dans lequel la matière inorganique est du carbonate de calcium.

3. Procédé de fabrication d'un article absorbant jetable (10), ledit article absorbant comprenant un film en matière plastique et/ou des fibres synthétiques, ledit procédé comprenant les étapes de :
a. réaction du CO₂ atmosphérique présentant un rapport ¹⁴C/¹²C supérieur ou égal à 1 x 10⁻¹³, de préférence supérieur ou égal à 3 x 10⁻¹³, de manière davantage préférée supérieur ou égal à 5 x 10⁻¹³ avec un sel métallique qui consiste en un sel d'un métal alcalin (Groupe 1) choisi parmi du lithium (Li), du sodium (Na) et du potassium (K) ou un sel d'un métal alcalino-terreux (Groupe 2) choisi parmi du magnésium (Mg) et du calcium (Ca) en présence d'eau afin d'obtenir un carbonate métallique consistant en un carbonate de métal alcalin ou un carbonate de métal alcalino-terreux,
b1. incorporation du carbonate métallique dans ledit film en matière plastique ou lesdites fibres synthétiques,
b2. incorporation dudit film en matière plastique ou desdites fibres synthétiques dans ledit article absorbant.

4. Procédé selon la revendication 3, dans lequel le sel métallique consiste en un sel de type sulfate métallique.

5. Procédé selon l'une quelconque des revendications 3 à 4, dans lequel le carbonate métallique consiste en du carbonate de calcium.
